# EUROPEAN PATENT APPLICATION

(11) **EP 4 397 248 A1**
(43) Date of publication of application: **10.07.2024**
(21) Application number: 23155491.6
(22) Date of filing: 08.02.2023
(51) Int. Cl.: A61B 10/00, G01N 33/72, G01N 1/40, A61B 10/02

(54) **MICRO RED BLOOD CELL COLLECTION DEVICE AND METHOD FOR COLLECTING AND DETECTING GLYCOSYLATED HEMOGLOBIN**

(30) Priority: 06.01.2023 US 202318093899
(71) Applicant: Anbio Biotechnology Limited, Road Town VG1110 Tortola (VG); Wang, Jincheng, Xiamen City Fujian (CN)
(72) Inventor: WANG, Daming, Xiamen City, Fujian Province (CN); WANG, Jincheng, Xiamen City, Fujian Province (CN)
(74) Representative: Sach, Greg Robert

(57) **Abstract**

The present application provides a micro red blood cell collection device for detecting glycosylated hemoglobin. By using the device, the extraction of packed red blood cells can be realized on one device by providing structures such as a blood-absorbing pad and a porous adhesive layer. The present application provides a method for collecting micro red blood cells. In the method, by using the device as a collection device, the micro red blood cells can be collected directly from the blood sample, thereby solving the defect of complicated operations required for sampling in the prior art. The present application provides a method for detecting glycosylated hemoglobin. In the method, by using the device to collect packed red blood cells and further directly detect glycosylated hemoglobin, the technical problem of inconvenient operation in the detection of glycosylated hemoglobin caused by the combined use of multiple devices in the prior art is solved.

## Description

### TECHNICAL FIELD

The present application relates to the field of red blood cell collection devices. Specifically, the present application relates to a micro red blood cell collection device and method for collecting and detecting glycosylated hemoglobin.

### BACKGROUND

Blood is composed of plasma and blood cells. The commonly used blood sample in clinical testing is plasma, but there are also items that require packed red blood cells as a test sample. For example, when biochemical testing of glycosylated hemoglobin, packed red blood cells are required as a test sample.

Glycosylated hemoglobin is the product of the combination of hemoglobin and blood sugar in red blood cells in human blood. Generally, the percentage of refined hemoglobin in total hemoglobin is used clinically as an indicator of whether the simple glycated hemoglobin content is normal. The percentage of refined hemoglobin in total hemoglobin can stably and reliably reflect the average blood sugar level of the previous 4 months, and this test will not be interfered by factors such as blood drawing time, fasting, insulin use, etc. It is the "gold standard" for diabetes monitoring in the world.

At present, common glycosylated hemoglobin detection kits (such as enzymatic method, immunoturbidimetric method, etc.) all need packed red blood cells formed by centrifugation or natural sedimentation of whole blood samples collected in vacuum blood collection tubes as detection objects, and calculate the percentage of glycosylated hemoglobin in the total hemoglobin by measuring the content of glycosylated hemoglobin in the packed red blood cell sample, so as to avoid the complicated detection and operation caused by the separate detection of total hemoglobin for each sample. Therefore, how to obtain packed red blood cells quickly and easily has become an important issue.

In the prior art, in order to obtain a fixed amount of packed red blood cells and further detect the percentage of glycosylated hemoglobin, three steps are usually required: blood collection, separation of packed red blood cells, and absorption of fixed packed red blood cells. Specifically, in the detection of the percentage of glycosylated hemoglobin, generally first use a vacuum blood collection tube to collect venous blood, or extract a certain volume of whole blood from the end of the human body into a container; and then, separate the red blood cells and plasma by centrifugation, natural sedimentation, filter element filtration, etc.; finally, absorb a fixed amount of packed red blood cells is absorbed by a pipetting device to detect the percentage of glycosylated hemoglobin.

However, the process of the detection method in the prior art is relatively complicated, time-consuming, and the cost is relatively high, and the amount of blood collected is relatively large. For example, centrifugation requires a special centrifuge, which needs to provide power for the centrifuge; and natural sedimentation, it takes hours of waiting time; At the same time, filtration requires a filter membrane of a special material, and the filtered red blood cells are not easy to take out for testing; similarly, the final collection of fixed packed red blood cells also needs to be transferred using a pipette. The whole process is time-consuming and laborious, and it is not easy to realize miniaturization and portability.

Therefore, the development of a miniaturized packed red blood cell collection device and a convenient packed red blood cell collection method has become the focus of people's research.

In view of this, this present application is proposed.

### SUMMARY OF THE APPLICATION

The first purpose of the present application is to provide a micro red blood cell collection device for detecting glycosylated hemoglobin. In the present application, the blood sample is directly absorbed and fixed through the blood-absorbing pad, thereby avoiding the use of blood collection containers; at the same time, in the present application, the blood-absorbing pad can be fixed by setting the porous adhesive layer, and at the same time, it will not affect the blood-absorbing pad to absorb blood samples. Further, the device of the present application can realize the extraction and fixation of packed red blood cells by simply removing the excess blood sample after drawing the blood sample, so that the extraction of packed red blood cells can be realized by using one device, thereby avoiding the technical problem that the miniaturization and convenience of the device cannot be realized due to the multi-device combination used in the prior art for extracting packed red blood cells.

The second purpose of the present application is to provide a method for collecting micro red blood cells. In the method of the present application, by using the device of the present application as the collection device, the micro red blood cells can be collected directly from the blood sample. This solves the technical problem of cumbersome operations caused by the use of multiple devices and multiple steps in the process of collecting packed red blood cells in the prior art. The method of the present application has the advantages of simple and fast operation.

The third purpose of the present application is to provide a method for detecting glycosylated hemoglobin. In the method of the present application, packed red blood cells are collected by using the device of the present application, and glycosylated hemoglobin is further directly detected. This solves the technical problem of inconvenient operation caused by the combined use of multiple devices in the detection process of glycosylated hemoglobin in the prior art. The method of the present application has the advantages of convenient operation method and accurate result.

In order to realize the above-mentioned purpose of the application, the application specially adopts following technical solution:

A micro red blood cell collection device for detecting glycosylated hemoglobin, comprising a fixing member, a blood-absorbing pad and a porous adhesive layer; the blood-absorbing pad is arranged at one end of the fixing member; the porous adhesive layer fixes the blood-absorbing pad on the fixing member by wrapping the blood-absorbing pad and providing with an end portion of the blood-absorbing pad.

In the present application, by providing the blood-absorbing pad and the porous adhesive layer covering it, the position of the blood- absorbing pad can be fixed, and the blood sample can be absorbed directly and the packed red blood cells can be further fixed. This avoids the multi-instrument and multi-step complex operations used in the extraction of packed red blood cells in the prior art. The device of the present application has the advantages of simple structure, convenient and fast detection and the like.

Optionally, in the present application, the length of the device is 3-10 cm.

Optionally, in the present application, the width of the device is 1.5-3.5 mm; preferably, in the present application, the width of the device is 2 mm.

Optionally, in the present application, the length of the blood-absorbing pad is shorter than the length of the base plate, and the length can be adjusted according to the amount of absorbed blood and the amount of further fixed packed red blood cells.

Optionally, in the present application, the hole size of the porous adhesive layer can be adjusted according to the amount of packed red blood cells to be absorbed and fixed.

Optionally, in the present application, the fixing member is a strip-shaped base plate, and the blood-absorbing pad is arranged on one side of the strip-shaped base plate and extends to its end. Further, one end of the porous adhesive layer is fixed on the side of the strip-shaped base plate, and the other end is fixed on the side opposite to the side where the blood-absorbing pad is arranged; and the width of the porous adhesive layer is the same as that of the strip-shaped base plate.

During the use of the device of the present application, by further adjusting and optimizing the structure of the base plate, the position of the porous adhesive layer, and the shape of the structure, the blood-absorbing pad can absorb blood samples from the uncoated side and the holes of the porous adhesive layer. This further improves sample uptake and fixation efficiency.

Optionally, in the present application, the device further comprises a blood removal device; wherein the blood removal device is arranged on the fixing member and is slidably connected with the fixing member.

In the present application, the blood removal device can further realize the removal of unfixed excess blood on the same device, and then realize the integrated device operation steps of packed red blood cell extraction, making the detection more convenient and quicker.

Optionally, in the present application, the blood removal device is connected to the fixing member through a clamping structure.

Optionally, in the present application, the blood removal device is a porous or membrane-like adsorption material.

In the present application, by using a porous or membrane-like material as the blood removal device, blood that is not fixed by the blood-absorbing pad can be fully removed, thereby improving the accuracy of detection.

Optionally, in the present application, the porous or membrane-like absorption material is one or a combination of polymer water-absorbing resin, cotton, absorption paper, and sponge.

In the present application, by further adjusting and optimizing of the materials used for the blood removal device, by using high-molecular water-absorbing resin with relatively excellent performance as the material of the blood removal device, and using the difference in blood absorption performance caused by the difference between the material of the blood removal device and the material of the blood-absorbing pad, the excess sample can be further removed, thereby further improving detection efficiency and accuracy.

Optionally, in the present application, the fixing member is a PET fixing member.

In the present application, the structural stability of the device of the present application is improved by selecting and optimizing the base plate material and using PET with excellent physical and mechanical properties as the base plate material.

Optionally, in the present application, the blood-absorbing pad is a glass fiber blood-absorbing pad.

In the application, the detection accuracy of the device of the application is improved by selecting and optimizing the material of the blood-absorbing pad and selecting glass fibers with good packing red blood cell fixation ability as the blood-absorbing pad material.

Optionally, in the present application, the porous adhesive layer is a porous waterproof adhesive tape.

In the present application, the structural stability of the device of the present application can be improved by selecting and optimizing the material of the porous adhesive layer and selecting a porous waterproof adhesive tape as the bonding layer that cannot absorb blood while having good adhesive ability. It will not affect the detection accuracy of the device of this application.

Optionally, the manufacturing method of the micro red blood cell collection device for detecting glycosylated hemoglobin according to the present application is as follows: Paste a 4mm wide glass fiber pad on the bottom of a rectangular PET board backing with a width of about 3~10cm, and then wrap the whole rectangular PET board and the end with the glass fiber pad with a porous waterproof adhesive tape, and ensure that the wrapped end is seamless, finally cut the whole board into strips with a width of 2mm, and obtain the device of the present application with a length of about 3-10cm and a width of about 2mm. Furthermore, the manufacturing method further includes the step of clamping the blood removal device in the middle of the base plate by a clamping structure. At the same time, the present application also provides a method for collecting micro red blood cells, in which the device described in the present application is used. Further, the method specifically includes the following steps: placing a structural part of the red blood cell collection device that is closely attached to the blood-absorbing pad into a blood sample for sample suction, and removing the blood sample not fixed by the blood-absorbing pad by squeezing and absorbing, and obtaining the collected micro red blood cells.

In the method of the present application, by using the device of the present application as the collection device, the micro red blood cells can be collected directly from the blood sample. This solves the technical problem of cumbersome operations caused by the use of multiple devices and multiple steps in the process of collecting packed red blood cells in the prior art. The method of the present application has the advantages of simple and fast operation.

Optionally, the method for collecting micro red blood cells described in the application is a method for collecting packed red blood cells; the collected micro red blood cells are packed red blood cells.

Further, the present application also provides a method for detecting glycosylated hemoglobin, using the device described in the application in the method. Further, the method includes the following steps: placing a structural part of the red blood cell collection device that is closely attached to the blood-absorbing pad into a blood sample for sample suction, and removing the blood sample not fixed by the blood-absorbing pad by squeezing and absorbing, and then placing the structural part in a hemoglobin detection reagent solution to detect glycosylated hemoglobin.

In the method of the present application, packed red blood cells are collected by using the device of the present application, and glycosylated hemoglobin is further directly detected. This solves the technical problem of inconvenient operation caused by the combined use of multiple devices in the detection process of glycosylated hemoglobin in the prior art. The method of the present application has the advantages of convenient operation method and accurate result.

Compared with the prior art, the beneficial effects of the present application are:
1) The device of this application has a simple structure, and can realize the absorption of blood samples and the extraction and fixation of packed red blood cells in the same device, so it has the advantages of convenient use, convenient and fast detection, etc.
2) In this application, the device of this application has good structural stability by selecting and optimizing the structural materials of each component in the device, and will not deform or fall off even if it is placed for a long time.
3) In the method of this application, by using the device of this application as a packed red blood cell collection device, it has the advantages of convenient operation and convenient use.

### BRIEF DESCRIPTION OF THE DRAWINGS

In order to more clearly illustrate the specific embodiments of the present application or the technical solutions in the prior art, the following will briefly introduce the accompanying drawings that need to be used in the description of the specific embodiments or prior art. Obviously, the drawings in the following description are some embodiments of the present application, and those skilled in the art can obtain other drawings according to these drawings without creative efforts.
Fig. 1 is a schematic structural diagram of the micro red blood cell collection device for detecting glycosylated hemoglobin provided by the application, 101-fixing member, 102-porous adhesive layer, 103-blood-absorbing pad;
Fig. 2 is a schematic structural diagram of a micro red blood cell collection device for detecting glycosylated hemoglobin provided by the present application, 101- fixing member, 102 - blood removal device, 103 - porous adhesive layer, and 104 - blood-absorbing pad.

### DETAILED DESCRIPTION

In order to make the purpose, technical solution and advantages of the present application clearer, the technical solution of the present application will be clearly and completely described below. Based on the specific implementation manners in this application, all other implementation manners obtained by a person skilled in the art without making creative efforts fall within the protection scope of this application.

In the description of the present application, it should be noted that the orientation or positional relationship indicated by terms "center", "upper", "lower", "left", "right", "vertical", "horizontal", "inner" and the like is based on the orientation or positional relationship shown in the accompanying drawings, only for the convenience of describing the application, rather than indicating or implying that the device or element referred to must have a specific orientation, be constructed in a specific orientation, and operate, and thus should not be construed as limiting the application. In addition, the terms "first", "second", and "third" are used for descriptive purposes only, and should not be construed as indicating or implying relative importance.

In the description of the present application, it should be noted that, unless otherwise specified and limited, the terms "installation", "connection" and "connected" should be understood in a broad sense. For example, it can be a fixed connection, a detachable connection, or an integral connection; it can be a mechanical connection or an electrical connection, it may be directly connected, or indirectly connected through an intermediary, and may be internally connected between two elements. A person skilled in the art can understand the specific meanings of the above terms in this application in specific situations.

### Embodiment 1

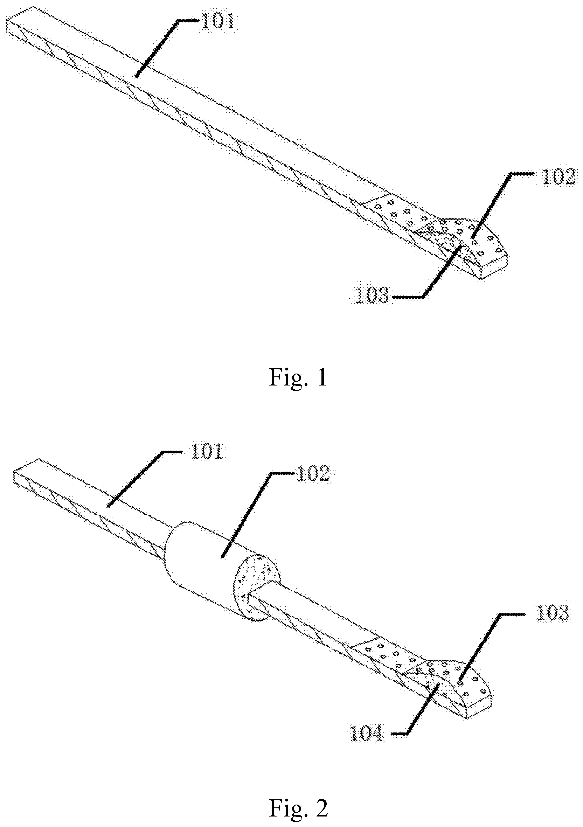

Please refer to FIG. 1, a micro red blood cell collection device for detecting glycosylated hemoglobin provided by the present application, comprises: a fixing member 101, a porous adhesive layer 102, and a blood-absorbing pad 103;

Wherein, the blood-absorbing pad 103 is arranged at one end of the fixing member 101; the porous adhesive layer102 fixes the blood-absorbing pad 103 on the fixing member 101 by wrapping the blood-absorbing pad 103 and providing with an end portion of the blood-absorbing pad 103.

### Embodiment 2

Please refer to FIG. 1, a micro red blood cell collection device for detecting glycosylated hemoglobin provided by the present application, comprises: a fixing member 101, a porous adhesive layer 102, and a blood-absorbing pad 103;
wherein the fixing member 101 is a strip-shaped base plate, and the blood-absorbing pad 103 is arranged on one side of the strip-shaped base plate and extends to its end;
at same time, one end of the porous adhesive layer 102 is fixed on the side of the strip-shaped base plate, and the other end is fixed on the side opposite to the side where the blood-absorbing pad 103 is arranged; and the width of the porous adhesive layer 102 is the same as that of the strip-shaped base plate.

Further, the fixing member 101 is a PET fixing member; the blood-absorbing pad 102 is a glass fiber blood-absorbing pad; the porous adhesive layer 103 is a porous waterproof adhesive tape.

### Embodiment 3

Please refer to FIG. 1, a micro red blood cell collection device for detecting glycosylated hemoglobin provided by the present application, comprises: a fixing member 101, a porous adhesive layer 102, and a blood-absorbing pad 103;

Wherein, the fixing member 101 is a strip-shaped base plate, and the blood-absorbing pad 103 is arranged on one side of the strip-shaped base plate and extends to its end;

At same time, one end of the porous adhesive layer 102 is fixed on the side of the strip-shaped base plate, and the other end is fixed on the side opposite to the side where the blood-absorbing pad 103 is arranged; and the width of the porous adhesive layer 102 is the same as that of the strip-shaped base plate.

Further, the fixing member 101 is a PET fixing member; the blood-absorbing pad 102 is a glass fiber blood-absorbing pad; the porous adhesive layer 103 is a porous waterproof adhesive tape.

At same time, the length of the base plate 101 is 3 cm, and the width of the base plate 101 is 2 mm.

### Embodiment 4

Please refer to Fig. 2, a micro red blood cell collection device for detecting glycosylated hemoglobin provided by the application, comprises: a fixing member 101, a blood removal device 102, a porous adhesive layer 103, a blood-absorbing pad 104;

Wherein, the blood-absorbing pad 104 is arranged at one end of the fixing member 101; the porous adhesive layer103 fixes the blood-absorbing pad 104 on the fixing member 101 by wrapping the blood-absorbing pad 104 and providing with an end portion of the blood-absorbing pad 104. The blood removal device 102 is arranged on the fixing member 101 and is slidably connected with the fixing member 101.

### Embodiment 5

Please refer to Fig. 2, a micro red blood cell collection device for detecting glycosylated hemoglobin provided by the application, comprises: a fixing member 101, a blood removal device 102, a porous adhesive layer 103, a blood-absorbing pad 104;
wherein the fixing member 101 is a strip-shaped base plate, and the blood-absorbing pad 104 is arranged on one side of the strip-shaped base plate and extends to its end; one end of the porous adhesive layer 103 is fixed on the side of the strip-shaped base plate, and the other end is fixed on the side opposite to the side where the blood-absorbing pad 104 is arranged; and the width of the porous adhesive layer 103 is the same as that of the strip-shaped base plate.

At same time, the blood removal device 102 is arranged on the fixing member 101 and is slidably connected with the fixing member101.

Further, the fixing member 101 is a PET fixing member; the blood removal device 102 is a sponge blood removal device, the porous adhesive layer 103 is a porous waterproof adhesive tape and the blood-absorbing pad 104 is a glass fiber blood-absorbing pad.

### Embodiment 6

Please refer to Fig. 2, a micro red blood cell collection device for detecting glycosylated hemoglobin provided by the application, comprises: a fixing member 101, a blood removal device 102, a porous adhesive layer 103, a blood-absorbing pad 104;
wherein the fixing member 101 is a strip-shaped base plate, and the blood-absorbing pad 104 is arranged on one side of the strip-shaped base plate and extends to its end; one end of the porous adhesive layer 103 is fixed on the side of the strip-shaped base plate, and the other end is fixed on the side opposite to the side where the blood-absorbing pad 104 is arranged; and the width of the porous adhesive layer 103 is the same as that of the strip-shaped base plate.

At same time, the blood removal device 102 is arranged on the fixing member 101 and is slidably connected with the fixing member 101.

Further, the fixing member 101 is a PET fixing member; the blood removal device 102 is a sponge blood removal device, the porous adhesive layer 103 is a porous waterproof adhesive tape and the blood-absorbing pad 104 is a glass fiber blood-absorbing pad.

Meanwhile, the length of the base plate 101 is 10 cm, and the width of the base plate 101 is 2 mm.

### Experimental example 1

Place the structural part with the blood-absorbing pad 103 of the micro red blood cell collection device for detecting glycosylated hemoglobin according to the embodiment 3 of the present application into the blood sample and carry out blood suction: then, take out the device, then squeeze the blood-absorbing pad 103 by using water-absorbing paper , absorb and remove the excess blood sample, and obtain the collected micro red blood cells, that is, packed red blood cells.

Then, soak the structural part of the device with the blood-absorbing pad 103 in the hemoglobin detection reagent solution, so that the packed red blood cells are released, so as to perform further detection of glycosylated hemoglobin.

### Experimental example 2

Place the structural part with the blood-absorbing pad 104 of the micro red blood cell collection device for detecting glycosylated hemoglobin according to the embodiment 6 of the present application into the blood sample and carry out blood suction: then, take out the device, and then move the blood removal device 102 to the blood-absorbing pad 104 through the clamping device, and squeeze the blood-absorbing pad 104 to remove excess blood samples, to obtain the collected micro red blood cells, that is, packed red blood cells .

Then, soak the structural part of the device with the blood-absorbing pad 104 in the hemoglobin detection reagent solution, so that the packed red blood cells are released, so as to perform further detection of glycosylated hemoglobin.

In the present application, the blood sample is effectively absorbed by the blood-absorbing pad, and the excess blood sample is removed by using the difference between the blood removal device and the blood-absorbing pad's blood-absorbing ability, thereby obtaining packed red blood cells, and detecting glycosylated hemoglobin. The application has the advantages of simple device structure, easy miniaturization, convenient operation and good accuracy of the detection method.

While particular embodiments of the application have been illustrated and described, it should be appreciated that various other changes and modifications could be made without departing from the spirit and scope of the application. It is therefore intended to cover in the appended claims all such changes and modifications that are within the scope of this application.

## Claims

1. A micro red blood cell collection device for detecting glycosylated hemoglobin, comprising a fixing member, a blood-absorbing pad and a porous adhesive layer; the blood-absorbing pad is arranged at one end of the fixing member; the porous adhesive layer fixes the blood-absorbing pad on the fixing member by wrapping the blood-absorbing pad and providing with an end portion of the blood-absorbing pad.

2. The device according to claim 1, wherein the fixing member is a strip-shaped base plate, and the blood-absorbing pad is arranged on one side of the strip-shaped base plate and extends to its end;
wherein one end of the porous adhesive layer is fixed on the side of the strip-shaped base plate, and the other end is fixed on the side opposite to the side where the blood-absorbing pad is arranged; and the width of the porous adhesive layer is the same as that of the strip-shaped base plate.

3. The device according to claim 1, wherein the device further comprises a blood removal device;
wherein the blood removal device is arranged on the fixing member and is slidably connected with the fixing member.

4. The device according to claim 3, wherein the blood removal device is a porous or membrane-like absorption material.

5. The device according to claim 4, wherein the porous or membrane-like absorption material is one or a combination of polymer water-absorbing resin, cotton, absorption paper, and sponge.

6. The device according to claim 1, wherein the fixing member is a PET fixing member.

7. The device according to claim 1, wherein the blood-absorbing pad is a glass fiber blood-absorbing pad.

8. The device according to claim 1, wherein the porous adhesive layer is a porous waterproof adhesive tape.

9. A method for collecting micro red blood cells, comprising the following steps: placing a structural part of the red blood cell collection device according to any one of claims 1-8 that is closely attached to the blood-absorbing pad into a blood sample for sample suction, and removing the blood sample not fixed by the blood-absorbing pad by squeezing and absorbing, and obtaining the collected micro red blood cells.

10. A method for detecting glycosylated hemoglobin, comprising the following steps: placing a structural part of the red blood cell collection device according to any one of claims 1-8 that is closely attached to the blood-absorbing pad into a blood sample for sample suction, and removing the blood sample not fixed by the blood-absorbing pad by squeezing and absorbing, and then placing the structural part in a hemoglobin detection reagent solution to detect glycosylated hemoglobin.
